# EUROPÄISCHE PATENTSCHRIFT

(11) **EP 3 478 231 B1**
(45) Veröffentlichungstag und Bekanntmachung des Hinweises auf die Patenterteilung: **12.08.2020**
(21) Anmeldenummer: 18734422.1
(22) Anmeldetag: 18.05.2018
(51) Int. Cl.: A61F 9/007, A61B 17/00

(54) **VORRICHTUNG ZUR BEREITSTELLUNG VON DRUCKLUFTIMPULSEN FÜR EIN OPHTHALMOLOGISCHES INSTRUMENT UND EIN STEUERUNGSVERFAHREN FÜR SOLCH EINE VORRICHTUNG**
DEVICE FOR PROVIDING COMPRESSED-AIR PULSES FOR AN OPHTHALMOLOGICAL INSTRUMENT AND A CONTROL METHOD FOR A DEVICE OF THIS KIND
DISPOSITIF POUR DÉLIVRER DES IMPULSIONS D'AIR COMPRIMÉ POUR UN INSTRUMENT OPHTALMOLOGIQUE ET PROCÉDÉ DE COMMANDE POUR UN TEL DISPOSITIF

(30) Priorität: 22.05.2017 DE 102017208584
(43) Veröffentlichungstag der Anmeldung: 08.05.2019
(73) Patentinhaber: Geuder AG, 69126 Heidelberg (DE)
(72) Erfinder: LOEW, Thomas, 68723 Schwetzingen (DE)
(74) Vertreter: Patent- und Rechtsanwälte Ullrich & Naumann
(86) Internationale Anmeldenummer: PCT/DE2018/200051
(87) Internationale Veröffentlichungsnummer: WO 2018/215032

(56) Entgegenhaltungen:
- WO-A1-2011/149621
- WO-A1-2015/164459
- US-A1- 2003 195 538

## Beschreibung

Die vorliegende Erfindung betrifft ein Steuerungsverfahren für eine Vorrichtung zur Bereitstellung von Druckluftimpulsen für ein ophthalmologisches Instrument, wobei ein Drucksystem über einen Druckluftgenerator mit Druck beaufschlagt wird und wobei ein Ventil des Drucksystems von einer Steuereinrichtung mittels eines Steuersignals betätigt wird, um die Druckluftimpulse zu erzeugen. Des Weiteren betrifft die Erfindung eine Vorrichtung zur Bereitstellung von Druckluftimpulsen für ein ophthalmologisches Instrument, mit einem Druckluftgenerator und einem Drucksystem, wobei das Drucksystem über den Druckluftgenerator mit Druckluft beaufschlagbar ist, wobei das Drucksystem ein Ventil aufweist und wobei das Ventil über ein von einer Steuereinrichtung erzeugtes Steuersignal betätigbar ist.

Verfahren und Vorrichtungen zur Bereitstellung von Druckluftimpulsen sind seit Jahren aus der Praxis bekannt und dienen beispielsweise zum Betrieb von Vitrektoren bzw. Cuttern, die im Rahmen einer Vitrektomie zum Entfernen von Teilen des Glaskörpers verwendet werden. Beispielhaft wird hierzu auf die DE 10 2008 019 790 A1 verwiesen. Darüber hinaus offenbart US 2003 195 53 ein Verfahren, wobei, je nach Druckluftimpulsrate, die Impulsparameter geändert werden; ferner wird das die Steuerung des Ventils in Abhängigkeit des Messwertes des Systemdrucks bestimmt.

Bei den bekannten Vitrektoren wird die eigentliche Schneideinrichtung durch ein vom Griff oder vom Kopf des Instruments abragendes Außenrohr und ein im Außenrohr verschiebbar geführtes, pneumatisch betätigbares Innenrohr gebildet. Im vorderen Bereich des Außenrohrs ist eine seitliche Absaugöffnung vorgesehen, deren Innenkanten mit den vorderen freien Außenkanten des Innenrohres schneidend bzw. scherend zusammenwirken. Wird der Vitrektor mit Druckluft beaufschlagt, wird das Innenrohr in Richtung des distalen Endes des Außenrohrs gedrückt und dadurch ein Schnitt ausgeführt. Bei der Entlüftung des Überdrucks wird mittels einer mechanischen Feder das Innenrohr wieder in die Ausgangslage verbracht.

Zur Auslenkung des Innenrohres werden Druckluftimpulse mit einer Frequenz von einigen Hertz bis hin zu einigen Kilohertz benötigt, wozu der Vitrektor über eine Leitung mit einer Vorrichtung zur Bereitstellung von Druckluftimpulsen verbunden ist. Eine solche Vorrichtung umfasst einen Druckluftgenerator, über den ein Drucksystem mit Druck beaufschlagbar ist. Das Drucksystem weist ein Ventil auf, so dass über eine Steuerung bzw. Schaltung des Ventils Druckluftimpulse erzeugbar sind, mit welchen das ophthalmologische Instrument betrieben wird. Das Steuersignal bzw. die Schaltimpulse des Ventils sind dabei für verschiedene Druckluftimpulsraten fest vorgegeben.

Bei der bekannten Vorrichtung ist problematisch, dass ein nahezu konstanter Steuerdruck vorliegen muss, um einen fehlerfreien Betrieb des Vitrektors zu gewährleisten. Erreicht der Druckluftgenerator seine Leistungsgrenze oder weist der Systemdruck zu hohe Schwankungen auf, führt dies zu Störungen in der Funktion des Vitrektors, bis hin zu einem Totalausfall.

Der vorliegenden Erfindung liegt daher die Aufgabe zugrunde, eine Vorrichtung der eingangs genannten Art sowie ein Steuerungsverfahren für eine solche Vorrichtung derart auszugestalten und weiterzubilden, dass eine sichere Funktion des ophthalmologischen Instruments auch bei schwankendem Systemdruck gewährleistet ist.

Erfindungsgemäß wird die voranstehende Aufgabe durch die Merkmale des Anspruches 1 gelöst. Danach ist das Steuerungsverfahren für eine Vorrichtung zur Bereitstellung von Druckluftimpulsen für ein ophthalmologisches Instrument, wobei ein Drucksystem über einen Druckluftgenerator mit Druck beaufschlagt wird und wobei ein Ventil des Drucksystems von einer Steuereinrichtung mittels eines Steuersignals betätigt wird, um die Druckluftimpulse zu erzeugen, dadurch gekennzeichnet, dass mit einem Drucksensor der herrschende Systemdruck gemessen wird und dass zumindest für die Erzeugung von Druckluftimpulsraten eines bestimmten Wertebereichs das Steuersignal von der Steuereinrichtung unter Berücksichtigung des Messwertes des Systemdrucks erzeugt wird.

In erfindungsgemäßer Weise ist zunächst erkannt worden, dass durch eine dynamische Ansteuerung des Ventils unter Berücksichtigung des herrschenden Systemdrucks die Funktion des Instruments auch bei schwankendem Systemdruck gewährleistet werden kann. Im Konkreten wird hierzu mit einem Drucksensor der Systemdruck bestimmt und zumindest bei bestimmten, insbesondere hohen, Druckluftimpulsraten zur Erzeugung des Steuersignals verwendet. Mit anderen Worten erfolgt die Erzeugung bzw. Berechnung des Steuersignals durch die Steuereinheit zumindest bereichsweise in Abhängigkeit des Systemdrucks. Es ist denkbar, dass die Bestimmung des Steuersignals unabhängig von der Druckluftimpulsrate stets unter Berücksichtigung des Messwertes des Systemdrucks erfolgen kann. Das Drucksystem kann Teil eines Druckluftkreises sein bzw. diesen bilden. Weiterhin kann das Drucksystem einen Druckluftspeicher aufweisen bzw. umfassen, der mittelbar oder unmittelbar durch den Druckluftgenerator mit Druckluft beaufschlagbar ist.

Durch das erfindungsgemäße Steuerungsverfahren ist es möglich, Störungen des Drucksystems, bspw. eine Leckage, sowie Leistungstoleranzen der einzelnen Komponenten der Vorrichtung auszugleichen. Des Weiteren ist ein Betrieb der Vorrichtung oberhalb der Leistungsgrenze des Kompressors möglich. Handelt es sich bei dem mit Druckluftimpulsen zu betreibenden Instrument um einen Vitrektor, wird durch die Druckluftimpulsrate die Schneidrate des Vitrektors bestimmt.

Der Begriff "Druckluft" ist nicht auf Umgebungsluft als Medium eingeschränkt. Vielmehr kann es sich dabei um ein beliebiges anderes Gas handeln.

Unter "Systemdruck" ist der Druck zu verstehen, der in dem Drucksystem bzw. dem Druckluftspeicher und ggf. in weiteren Bauteilen der Vorrichtung herrscht.

Bei der "Steuereinrichtung" kann es sich beispielsweise um einen Mikrokontroller oder eine andere geeignete Recheneinrichtung handeln.

Für Druckluftimpulsraten von weniger als 500 Druckluftimpulsen pro Minute wird als Steuersignal ein fester Wert für die Öffnungszeit des Ventils und somit eine feste Impulsbreite vorgegeben.

Für mittlere Druckluftimpulsraten bzw. Schneidraten des Instruments wird durch das Steuersignal ein festes Verhältnis zwischen Öffnungszeit und Schließzeit des Ventils vorgegeben werden. Dabei handelt es sich um Druckluftimpulsraten von 500 bis 1500 Druckluftimpulsen pro Minute. Gemäß einer besonders einfachen Ausgestaltung kann als Steuersignal ein fester Wert für das Verhältnis zwischen Öffnungszeit und Schließzeit des Ventils und somit ein fixer Duty-Cycle des Ventils vorgegeben sein. Für hohe Druckluftimpulsraten wird das Steuersignal von der Steuereinrichtung in Abhängigkeit des gemessenen Systemdrucks bestimmt. Hierbei handelt es sich um Druckluftimpulsraten von über 1500 Druckluftimpulsen pro Minute. Durch diese Maßnahme kann eine Korrektur des Verhältnisses zwischen der Öffnungszeit des Ventils und der Schließzeit des Ventils in Abhängigkeit von dem Systemdruck realisiert werden. Insbesondere ist denkbar, dass bei diesen hohen Druckluftimpulsraten das Verhältnis y zwischen Öffnungs- und Schließzeit des Ventils durch eine mathematische Funktion in Abhängigkeit des gemessenen Systemdrucks x berechnet wird, beispielsweise mittels der linearen Funktion y = - a•x +b, wobei a und b konstante Werte sind. Die Verwendung anderer mathematischer Funktionen ist möglich, beispielsweise einer Exponentialfunktion.

Es wird ausdrücklich darauf hingewiesen, dass denkbar ist, dass die Erzeugung des Steuersignals unabhängig von der Druckluftimpulsrate stets unter Berücksichtigung des Messwertes des Systemdrucks erfolgen kann. Alternativ oder zusätzlich kann eine Optimierung des Duty-Cycle-Managements des Vitrektors und/oder des Laufverhaltens des Vitrektors durch verschiedene mathematische Berechnungsformeln, bspw. eine Exponentialkurve, erfolgen.

Des Weiteren ist es von Vorteil, wenn die Druckluftimpulsrate über ein Eingabemittel veränderbar und/oder einstellbar ist. Somit kann von einem Operateur beispielsweise die Schneidrate des Instruments bzw. Vitrektors eingestellt werden bzw. ein Sollwert der Druckluftimpulsrate definiert werden.

In besonders vorteilhafter Weise wird die Druckluftimpulsrate schrittweise angepasst, wenn der Betrag der Differenz zwischen Sollwert und Istwert der Druckluftimpulsrate einen definierten Wert überschreitet (Sollwertsprung). Durch diesen "Sanftanlauf" wird eine große Änderung der Druckluftimpulsrate, die beispielsweise durch den Operateur durch das Eingabemittel vorgegeben wird, nicht schlagartig vorgenommen, sondern verteilt über mehrere Druckluftimpulse angepasst. Dadurch wird das Laufverhalten des Instruments, beispielsweise des Vitrektors, bei Sollwertsprüngen erheblich verbessert.

Um eine besonders einfache Konstruktion und zuverlässige Messung des Systemdrucks zu erreichen, ist es denkbar, dass der in dem Druckluftspeicher des Drucksystems herrschende Druck gemessen wird. Dadurch wird eine Verfälschung des Messwertes durch vom Betrieb des Druckluftgenerators und/oder des Ventils hervorgerufene Störsignale minimiert. Der hierzu verwendete Drucksensor kann in idealer Weise eine erhöhte Medienverträglichkeit aufweisen und robust sein, so dass Staub und Abrieb des Druckluftgenerators bzw. Kompressors sowie eine erhöhte Luftfeuchtigkeit bzw. Kondenswasser durch das Verdichten der Luft die Funktionsfähigkeit des Sensors nicht oder allenfalls vernachlässigbar beeinflussen.

Die zugrunde liegende Aufgabe wird des Weiteren durch die Merkmale von Anspruch 9 gelöst. Danach ist eine Vorrichtung zur Bereitstellung von Druckluftimpulsen für ein ophthalmologisches Instrument, mit einem Druckluftgenerator und einem Drucksystem, wobei das Drucksystem über den Druckluftgenerator mit Druckluft beaufschlagbar ist, wobei das Drucksystem ein Ventil aufweist und wobei das Ventil über ein von einer Steuereinrichtung erzeugtes Steuersignal betätigbar ist, dadurch gekennzeichnet, dass ein Drucksensor zur Messung des herrschenden Systemdrucks angeordnet ist und dass der Messwert des Systemdrucks von der Steuereinrichtung abrufbar ist.

In erfindungsgemäßer Weise ist erkannt worden, dass die zugrunde liegende Aufgabe durch eine Berücksichtigung des Systemdrucks zumindest bei der Erzeugung von bestimmten Druckluftimpulsraten auf verblüffend einfache Weise lösbar ist. Dazu weist die Vorrichtung einen Drucksensor auf, mittels welchem der Systemdruck bestimmbar ist. Der Systemdruck kann somit von der Steuereinrichtung bei der Erzeugung des Steuersignals zur Betätigung des Ventils berücksichtigt werden. Folglich ist der Begriff "abrufbar" dahingehend zu verstehen, dass der gemessene Wert des Systemdrucks der Steuereinrichtung zuführbar bzw. von dieser erfassbar ist, so dass unter Berücksichtigung des Systemdrucks die Steuerung des Ventils erfolgen kann.

Um die Druckluftimpulsrate und somit die Schnittrate eines mit der Vorrichtung verbundenen Vitrektors einzustellen, kann ein Eingabemittel vorgesehen sein. Dabei kann es sich beispielsweise um ein Fußpedal bzw. Fußschalter, einen Touchscreen, eine Tastatur etc. handeln.

In weiter vorteilhafter Weise erbringt der Druckluftgenerator eine Luftleistung von 12 Liter pro Minute bei einem Druck 2 bar bis 3 bar, insbesondere von 2,2 bar bis 2,8 bar, vorzugsweise von 2,4 bar.

Es wird darauf hingewiesen, dass die zuvor erörterten Merkmale zu dem erfindungsgemäßen Steuerungsverfahren auch eine vorrichtungsgemäße Ausprägung haben können. Eine Kombination dieser Merkmale mit den den Vorrichtungsanspruch betreffenden Merkmalen ist nicht nur möglich, sondern von Vorteil und ausdrücklich Teil der Offenbarung. Das erfindungsgemäße Verfahren kann somit Merkmale der Ansprüche 1 bis 8, der entsprechenden Beschreibung sowie der nachstehenden Figurenbeschreibung aufweisen. Auch kann das erfindungsgemäße Steuerungsverfahren Merkmale der erfindungsgemäßen Vorrichtung aufweisen.

Es gibt nun verschiedene Möglichkeiten, die Lehre der vorliegenden Erfindung in vorteilhafter Weise auszugestalten und weiterzubilden. Dazu ist einerseits auf die dem Anspruch 1 sowie dem Anspruch 9 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Darstellung eine aus dem Stand der Technik bekannte Vorrichtung zur Bereitstellung von Druckluftimpulsen für ein ophthalmologisches Instrument,
- Fig. 2: in einer schematischen Darstellung eine erfindungsgemäße Vorrichtung, anhand der das erfindungsgemäße Steuerungsverfahren darstellbar ist.

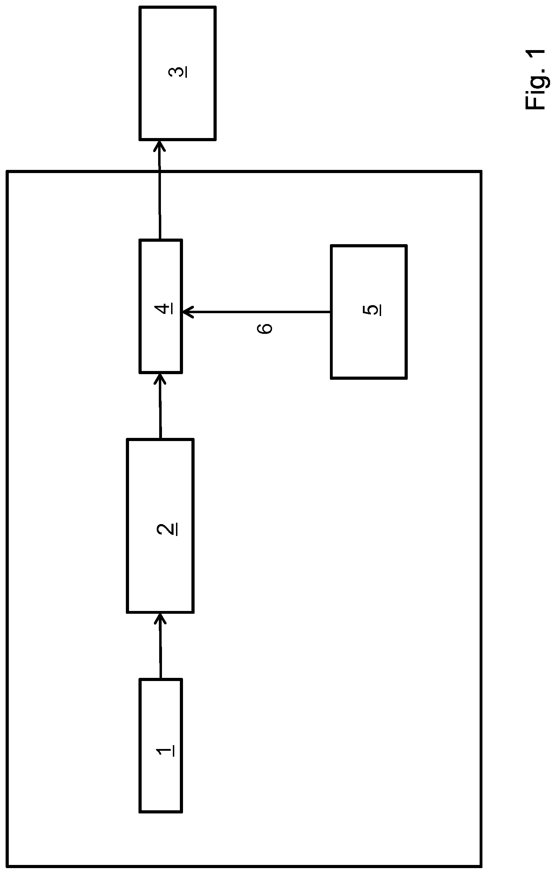

Fig. 1 zeigt in einer schematischen Darstellung eine aus dem Stand der Technik bekannte Vorrichtung. Die Vorrichtung umfasst einen Druckluftgenerator 1 und ein Drucksystem 2. Über den Druckluftgenerator 1 ist das Drucksystem 2 mit Druckluft beaufschlagbar. Des Weiteren ist ein Instrument 3 über eine Fluidleitung und ein Ventil 4 mit dem Drucksystem 2 verbunden. Zur Erzeugung der benötigten Druckluftimpulse, wird das Ventil 4 über eine Steuereinrichtung 5 durch ein Steuersignal 6 betätigt, d.h. geöffnet bzw. geschlossen. Beispielsweise kann das Instrument 3 als Vitrektor ausgebildet sein, der durch die Druckluftimpulse eine Schneidbewegung ausführt, um Gewebe aus einem Auge zu entfernen.

Für das Steuersignal 6, d.h. für die Schaltimpulse des Ventils 4, sind feste Werte hinterlegt, so dass ein konstanter Druck für einen fehlerfreien Betrieb der Vorrichtung notwendig ist. Erlangt der Druckluftgenerator 1 seine Leistungsgrenze oder weist der Systemdruck zu hohe Schwankungen auf, treten Störungen im Betrieb des Vitrektors auf, bis hin zu einem Totalausfall.

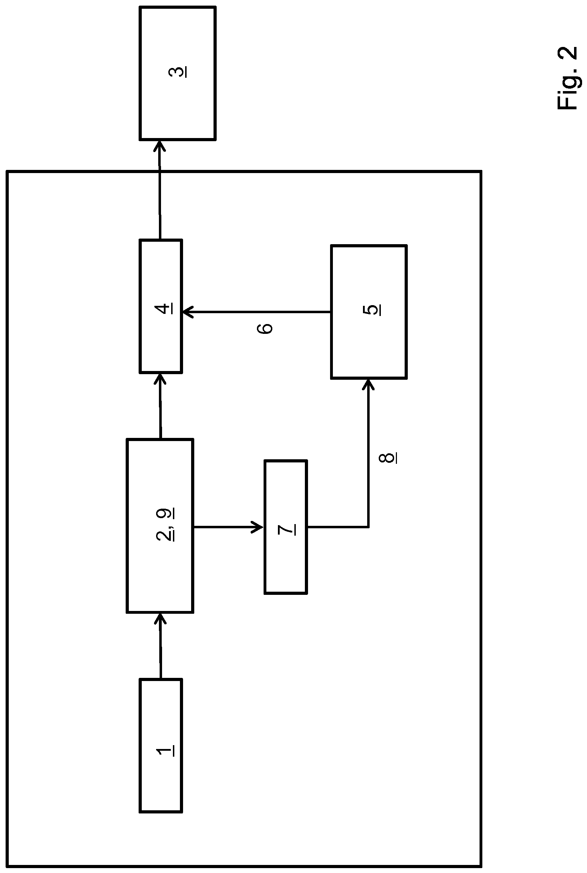

Fig. 2 zeigt in einer schematischen Darstellung ein Ausführungsbeispiel einer erfindungsgemäßen Vorrichtung, anhand der das erfindungsgemäße Steuerungsverfahren beschreibbar ist.

Die Vorrichtung umfasst einen Druckluftgenerator 1, der mit einem Drucksystem 2 wirkverbunden ist, so dass das Drucksystem 2 mit Druck bzw. Druckluft beaufschlagbar ist. Das Drucksystem 2 kann Teil eines Druckluftkreises sein bzw. diesen bilden und einen Druckluftspeicher 9 sowie ein Ventil 4 aufweisen, über das Druckluftimpulse erzeugbar sind, mit welchen das Instrument 3 betreibbar ist.

Aus Fig. 2 geht des Weiteren hervor, dass ein Drucksensor 7 angeordnet ist, mit dem der Systemdruck, hier der Druck in dem Drucksystem 2 bzw. dem Druckluftspeicher 9, messbar ist. Der Messwert 8 des Systemdrucks wird an die Steuereinrichtung 5 übermittelt bzw. ist von dieser abrufbar. Somit kann die Erzeugung des Steuersignals 6 zur Schaltung des Ventils 4 unter Berücksichtigung des gemessenen Systemdrucks erfolgen. Hierdurch ist eine dynamische Berechnung des Steuersignals 6 möglich, wodurch ein störungsfreier Betrieb eines Instruments 3 bzw. Vitrektors auch bei schwankendem Systemdruck sowie ein Betrieb oberhalb der Leistungsgrenze möglich ist.

Idealer Weise wird das Instrument 3 bzw. der Vitrektor mit einem festen Druck beaufschlagt, der während der Öffnungszeit des Ventils 4 an dem Instrument 3 anliegt, beispielsweise 2,4 bar. Weist der Druckluftgenerator 1 bzw. Kompressor eine Luftleistung von 12 Liter pro Minute bei einem Druck von 2,4 bar auf, so folgt daraus, dass aus einem geregelten Druckluftkreis (mit diesem Druckluftgenerator 1) bei 2,4 bar Nenndruck höchstens 12 Liter pro Minute an Druckluft entnommen werden kann. Dies bildet die Leistungsgrenze dieses Druckluftgenerators 1. Sobald mehr Luft entnommen wird, fällt der Systemdruck unter den Nenndruck, da der Druckluftgenerator 1 bei diesem Luftverbrauch den geforderten Druck nicht mehr aufbringen kann.

Mit zunehmender Druckluftimpulsrate bzw. Schneidrate des Vitrektors 3 nimmt der Luftverbrauch zu. Ist der Luftverbrauch höher als die Luftleistung des Druckluftgenerators 1 bei Nenndruck, d. h. besteht ein Betrieb oberhalb der Leistungsgrenze, sinkt der Systemdruck unter der Nenndruck ab und der Arbeitspunkt des Vitrektors 3 verschiebt sich. Durch die erfindungsgemäße Vorrichtung und das erfindungsgemäße Steuerungsverfahren ist es möglich, die Öffnungs- und Schließzeiten des Ventils 4 an die Verschiebung des Arbeitspunktes anzupassen bzw. diese auszugleichen.

Beispielsweise kann bei dem erfindungsgemäßen Steuerungsverfahren in einem Bereich geringer und/oder mittlerer Druckluftimpulsraten bzw. Schneidraten das Steuersignal 6 aus einer Lookup-Tabelle von der Steuereinrichtung 5 abgerufen werden. Bei hohen Druckluftimpulsraten und somit Schneidraten des Vitrektors 3 kann die Erzeugung des Steuersignals in erfindungsgemäßer Weise unter Berücksichtigung des Messwertes 8 des Systemdrucks erfolgen. Des Weiteren ist denkbar, dass die Erzeugung des Steuersignals 6 unabhängig von der Druckluftimpulsrate stets unter Berücksichtigung des Messwertes 8 ermittelt wird.

Hinsichtlich weiterer vorteilhafter Ausgestaltungen der erfindungsgemäßen Vorrichtung wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele der erfindungsgemäßen Vorrichtung lediglich zur Erörterung der beanspruchten Lehre dienen, diese jedoch nicht auf die Ausführungsbeispiele einschränken. Die Erfindung wird in den Ansprüchen definiert; andere Ausführungsformen sind lediglich für illustrative Zwecke offenbart.

### Bezugszeichenliste

- 1: Druckluftgenerator
- 2: Drucksystem
- 3: Instrument
- 4: Ventil
- 5: Steuereinrichtung
- 6: Steuersignal
- 7: Drucksensor
- 8: Messwert (Systemdruck)
- 9: Druckluftspeicher

## Patentansprüche

1. Steuerungsverfahren für eine Vorrichtung zur Bereitstellung von Druckluftimpulsen für ein ophthalmologisches Instrument (3), wobei ein Drucksystem (2) über einen Druckluftgenerator (1) mit Druck beaufschlagt wird und wobei ein Ventil (4) des Drucksystems (2) von einer Steuereinrichtung (5) mittels eines Steuersignals (6) betätigt wird, um die Druckluftimpulse zu erzeugen, wobei mit einem Drucksensor (7) der herrschende Systemdruck gemessen wird und dass zumindest für die Erzeugung von Druckluftimpulsraten eines bestimmten Wertebereichs das Steuersignal (6) von der Steuereinrichtung (5) unter Berücksichtigung des Messwertes (8) des Systemdrucks erzeugt wird,
**dadurch gekennzeichnet, dass** für geringe Druckluftimpulsraten von weniger als 500 Druckluftimpulsen pro Minute, das Steuersignal (6) eine Öffnungszeit des Ventils (4) vorgibt,
dass für mittlere Druckluftimpulsraten von 500 und 1500 Druckluftimpulsen pro Minute das Steuersignal (6) ein festes Verhältnis zwischen der Öffnungszeit und der Schließzeit des Ventils (4) vorgibt und
dass für hohe Druckluftimpulsraten von über 1500 Druckluftimpulsen pro Minute, das Steuersignal (6) von der Steuereinrichtung (5) in Abhängigkeit des Messwertes (8) des Systemdrucks bestimmt wird.

2. Steuerungsverfahren nach Anspruch 1, **dadurch gekennzeichnet, dass**, bei hohen Druckluftimpulsraten, das Verhältnis zwischen Öffnungs- und Schließzeit des Ventils (4) durch eine mathematische Funktion in Abhängigkeit des Messwertes (8) berechnet wird.

3. Steuerungsverfahren nach Anspruch 1 oder 2, **dadurch gekennzeichnet, dass** ein Sollwert der Druckluftimpulsrate über ein Eingabemittel veränderbar und/oder einstellbar ist.

4. Steuerungsverfahren nach dem Anspruch 3, **dadurch gekennzeichnet, dass** die Druckluftimpulsrate schrittweise angepasst wird, wenn der Betrag der Differenz zwischen Sollwert und Istwert der Druckluftimpulsrate einen definierten Wert überschreitet.

5. Steuerungsverfahren nach einem der Ansprüche 1 bis 4, **dadurch gekennzeichnet, dass** zur Bestimmung des Systemdrucks der in dem Drucksystem (2) herrschende Druck gemessen wird.

6. Vorrichtung zur Bereitstellung von Druckluftimpulsen für ein ophthalmologisches Instrument (3), mit einem Druckluftgenerator (1) und einem Drucksystem (2), wobei das Drucksystem (2) über den Druckluftgenerator (1) mit Druckluft beaufschlagbar ist, wobei das Drucksystem (2) ein Ventil (4) aufweist und wobei das Ventil (4) über ein von einer Steuereinrichtung (5) erzeugtes Steuersignal (6) betätigbar ist,
**dadurch gekennzeichnet, dass** ein Drucksensor (7) zur Messung des herrschenden Systemdrucks angeordnet ist und dass der Messwert (8) des Systemdrucks von der Steuereinrichtung (5) abrufbar ist, wobei für geringe Druckluftimpulsraten von weniger als 500 Druckluftimpulsen pro Minute, über das Steuersignal (6) eine Öffnungszeit des Ventils (4) vorgebbar ist,
dass für mittlere Druckluftimpulsraten von 500 und 1500 Druckluftimpulsen pro Minute das Steuersignal (6) ein festes Verhältnis zwischen der Öffnungszeit und der Schließzeit des Ventils (4) vergebbar ist und
dass für hohe Druckluftimpulsraten von über 1500 Druckluftimpulsen pro Minute, das Steuersignal (6) von der Steuereinrichtung (5) in Abhängigkeit des Messwertes (8) des Systemdrucks bestimmbar ist.

7. Vorrichtung nach Anspruch 6, **dadurch gekennzeichnet, dass** ein Eingabemittel angeordnet ist, um eine Druckluftimpulsrate einzustellen.

8. Vorrichtung nach Anspruch 6 oder 7, **dadurch gekennzeichnet, dass** der Druckluftgenerator (1) eine Luftleistung von 12 Liter pro Minute bei einem Druck 2 bar bis 3 bar, insbesondere von 2,2 bar bis 2,8 bar, vorzugsweise von 2,4 bar, erbringt.

## Claims

1. Control method for a device for providing compressed air pulses for an ophthalmological instrument (3), wherein a pressure system (2) is acted on with pressure by means of a compressed air generator (1) and wherein a valve (4) of the pressure system (2) is activated by a control device (5) by means of a control signal (6) in order to produce the compressed air pulses, wherein using a pressure sensor (7) the applied system pressure is measured and wherein at least for the production of compressed air pulse rates of a specific value range the control signal (6) is produced by the control device (5) taking into account the measurement value (8) of the system pressure,
**characterised in that**, for small compressed air pulse rates of less than 500 compressed air pulses per minute, the control signal (6) predetermines an opening time of the valve (4),
**in that**, for mean compressed air pulse rates of 500 and 1500 compressed air pulses per minute, the control signal (6) predetermines a fixed ratio between the opening time and the closing time of the valve (4), and
**in that**, for high compressed air pulse rates of more than 1500 compressed air pulses per minute, the control signal 6 is determined by the control device (5) in accordance with the measurement value (8) of the system pressure.

2. Control method according to claim 1, **characterised in that**, at high compressed air rates, the ratio between the opening and closing time of the valve (4) is calculated by means of a mathematical function in accordance with the measurement value (8).

3. Control method according to claim 1 or claim 2, **characterised in that** a desired value of the compressed air pulse rate can be changed and/or adjusted via an input means.

4. Control method according to claim 3, **characterised in that** the compressed air pulse rate is adapted gradually when the value of the difference between the desired value and actual value of the compressed air pulse rate exceeds a defined value.

5. Control method according to any one of claims 1 to 4, **characterised in that**, in order to determine the system pressure, the pressure applied in the pressure system (2) is measured.

6. Device for providing compressed air pulses for an ophthalmological instrument (3), having a compressed air generator (1) and a pressure system (2), wherein the pressure system (2) can be acted on with compressed air via the compressed air generator (1), wherein the pressure system (2) has a valve (4) and wherein the valve (4) can be activated by means of a control signal (6) which is produced by a control device (5),
**characterised in that** a pressure sensor (7) for measuring the applied system pressure is arranged, and **in that** the measurement value (8) of the system pressure can be retrieved by the control device (5), wherein, for small compressed air pulse rates of less than 500 compressed air pulses per minute, an opening time of the valve (4) can be predetermined via the control signal (6),
**in that**, for mean compressed air pulse rates of 500 and 1500 compressed air pulses per minute, a fixed ratio between the opening time and the closing time of the valve (4) can be predetermined via the control signal (6), and
**in that** for high compressed air pulse rates of more than 1500 compressed air pulses per minute, the control signal (6) can be determined by the control device (5) in accordance with the measurement value (8) of the system pressure.

7. Device according to claim 6, **characterised in that** an input means is arranged in order to adjust a compressed air pulse rate.

8. Device according to claim 6 or claim 7, **characterised in that** the compressed air generator (1) produces an air capacity of 12 litres per minute at a pressure of from 2 bar to 3 bar, in particular from 2.2 bar to 2.8 bar, preferably of 2.4 bar.

## Revendications

1. Procédé de commande pour un dispositif de production d'impulsions d'air comprimé pour un instrument ophtalmologique (3), dans lequel un système de pression (2) peut être alimenté en pression par l'intermédiaire d'un générateur d'air comprimé (1) et dans lequel une soupape (4) du système de pression (2) est actionnée par un dispositif de commande (5) au moyen d'un signal de commande (6), afin de générer les impulsions d'air comprimé, dans lequel, avec un capteur de pression (7), la pression régnant dans le système est mesurée et, au moins pour la production de fréquences d'impulsions d'air comprimé d'un intervalle de valeurs déterminé, le signal de commande (6) est généré par le dispositif de commande (5) en tenant compte de la valeur de mesure (8) de la pression du système,
**caractérisé en ce que**, pour de faibles fréquences d'impulsions d'air comprimé inférieures à 500 impulsions d'air comprimé par minute, le signal de commande (6) impose un temps d'ouverture de la soupape (4),
pour des fréquences d'impulsions d'air comprimé moyennes de 500 et 1500 impulsions d'air comprimé par minute, le signal de commande (6) impose un rapport fixe entre le temps d'ouverture et le temps de fermeture de la soupape (4) et
pour des fréquences d'impulsions d'air comprimé élevées supérieures à 1500 impulsions d'air comprimé par minute, le signal de commande (6) est déterminé par le dispositif de commande (5) en fonction de la valeur de mesure (8) de la pression du système.

2. Procédé de commande selon la revendication 1, **caractérisé en ce que**, lors de fréquences d'impulsions d'air comprimé élevées, le rapport entre le temps d'ouverture et le temps de fermeture de la soupape (4) est calculé par une fonction mathématique en fonction de la valeur de mesure (8).

3. Procédé de commande selon la revendication 1 ou 2, **caractérisé en ce qu'**une valeur de consigne de la fréquence d'impulsions d'air comprimé peut être modifiée et/ou réglée par l'intermédiaire d'un moyen de saisie.

4. Procédé de commande selon la revendication 3, **caractérisé en ce que** la fréquence d'impulsions d'air comprimé est adaptée pas-à-pas lorsque la valeur absolue de la différence entre la valeur de consigne et la valeur réelle de la fréquence d'impulsions d'air comprimé dépasse une valeur définie.

5. Procédé de commande selon l'une des revendications 1 à 4, **caractérisé en ce que**, pour la détermination de la pression du système, la pression régnant dans le système de pression (2) est mesurée.

6. Dispositif pour la production d'impulsions d'air comprimé pour un instrument ophtalmologique (3), avec un générateur d'air comprimé (1) et un système de pression (2) dans lequel le système de pression (2) peut être alimenté en air comprimé par l'intermédiaire du générateur d'air comprimé (1), dans lequel le système de pression (2) comprend une soupape (4) et dans lequel la soupape (4) peut être actionnée par l'intermédiaire d'un signal de commande (6) généré par un dispositif de commande (5),
**caractérisé en ce qu'**un capteur de pression (7) est prévu pour la mesure de la pression régnant dans le système de pression et **en ce que** la valeur de mesure (8) de la pression du système peut être interrogée par le dispositif de commande (5), dans lequel, pour de faibles fréquences d'impulsions d'air comprimé inférieures à 500 impulsions d'air comprimé par minute, un temps d'ouverture de la soupape (4) peut être imposé par l'intermédiaire du signal de commande (6),
pour des fréquences d'impulsions d'air comprimé moyennes de 500 et 1500 impulsions d'air comprimé par minute, un rapport fixe entre le temps d'ouverture et le temps de fermeture de la soupape (4) peut être imposé par l'intermédiaire du signal de commande (6) et
pour des fréquences d'impulsions d'air comprimé élevées supérieures à 1500 impulsions d'air comprimé par minute, le signal de commande (6) peut être déterminé par le dispositif de commande (5) en fonction de la valeur de mesure (8) de la pression du système.

7. Dispositif selon la revendication 6, **caractérisé en ce qu'**un moyen de saisie est disposé pour régler une fréquence d'impulsions d'air comprimé.

8. Dispositif selon la revendication 6 ou 7, **caractérisé en ce que** le générateur d'air comprimé (1) fournit un débit d'air de 12 litres par minute à une pression de 2 bar à 3 bars, plus particulièrement de 2,2 bars à 2,8 bars, de préférence de 2,4 bars.
